(19) Europäisches Patentamt / European Patent Office / Office européen des brevets

(11) **EP 1 888 142 B1**

(12) **EUROPEAN PATENT SPECIFICATION**

(45) Date of publication and mention of the grant of the patent:
**24.10.2018 Bulletin 2018/43**

(21) Application number: **06772785.9**

(22) Date of filing: **09.06.2006**

(51) Int Cl.:
*A61M 1/10* (2006.01)          *H02K 33/12* (2006.01)
*A61M 1/12* (2006.01)

(86) International application number:
**PCT/US2006/022607**

(87) International publication number:
**WO 2006/135802 (21.12.2006 Gazette 2006/51)**

(54) **ELECTROMAGNETIC DRIVE FOR A VENTRICULAR ASSIST DEVICE**

ELEKTROMAGNETISCHER ANTRIEB FÜR EIN VENTRIKULÄRES HILFSSYSTEM

COMMANDE ELECTROMAGNETIQUE POUR DISPOSITIF D'ASSISTANCE VENTRICULAIRE

(84) Designated Contracting States:
**DE FR GB IT**

(30) Priority: **09.06.2005 US 689617 P**

(43) Date of publication of application:
**20.02.2008 Bulletin 2008/08**

(73) Proprietors:
• **World Heart Corporation**
  **Oakland, California 94621 (US)**
• **Jassawalla, Jal**
  **Orinda, California 94563 (US)**

(72) Inventors:
• **MILLER, Phillip, J.**
  **Berkeley, California 94708 (US)**
• **LAFORGE, David, H.**
  **Kensington, California 94708 (US)**

• **JASSAWALLA, Jal**
  **Orinda, California 94563 (US)**

(74) Representative: **HGF Limited**
  **4th Floor**
  **Merchant Exchange**
  **17-19 Whitworth Street West**
  **Manchester M1 5WG (GB)**

(56) References cited:
  **DE-A1- 3 804 768       US-A- 2 323 441**
  **US-A- 4 565 497       US-A- 4 756 302**
  **US-B1- 6 264 601       US-B1- 6 342 746**
  **US-B1- 6 758 657       US-B2- 6 608 539**
  **US-B2- 6 616 413       US-B2- 6 969 345**

• **ZAREBA K.M.: 'The Artificial Heart - Past, Present, and Future' MED. SCI. MONIT. vol. 8, no. 3, 2002, pages RA72 - RA77, XP003014405**

**Description**

**FIELD OF THE INVENTION**

The present disclosure relates to devices and associated methods for pumping blood. More particularly, the present disclosure relates to a drive for use in ventricular assist devices that assist or replace the function of one or both ventricles of the heart.

**BACKGROUND OF THE INVENTION**

The American Heart Association estimates that there are approximately 5 million people with congestive heart failure in the United States and 550,000 new cases diagnosed annually. Those numbers will only rise in the foreseeable future with the aging of the baby-boom generation. According to the Framingham Heart Study, the five-year mortality rate for patients with congestive heart failure was 75 percent in men and 62 percent in women. Standard medical and surgical therapies benefit only a small percentage of patients with ventricular dysfunction. Potential cardiac transplant recipients with hemodynamic instability may receive temporary mechanical circulatory support, such as an implantable blood pump, as a bridge to cardiac transplantation. Moreover, estimates in the field suggest that 17,000 to 66,000 patients each year in the United States may benefit from a permanent blood pump.

The ventricular assist device (VAD) is a blood pump designed to assist or replace the function of either ventricle, or both ventricles, of the heart. A right ventricular assist device (RVAD) supports pulmonary circulation by receiving or withdrawing blood from the right ventricle and returning it to the pulmonary artery. A left ventricular assist device (LVAD) supports systemic perfusion by receiving or withdrawing blood from the left ventricle (or left atrium) and returning it to the aorta. A biventricular assist device (BVAD) supports both ventricles of the heart. Ventricular assist devices may be either implantable or extracorporeal, with implantable VADs positioned intracorporeally in the anterior abdominal wall or within a body cavity (other than the pericardium) and with extracorporeal VADs located paracorporeally, along the patient's anterior abdominal wall, or externally at the patient's bedside.

The first ventricular assist devices attempted to mimic the pulsatile flow of the natural left ventricle (LV) by utilizing flexible chambers with volumes approximately equal to the volume of the respective ventricle being assisted. The typical volume of blood expelled by the left ventricle of an adult is between 70-90 ml, but may range from 40-120 ml. The chambers are expanded and contracted, much like a natural ventricle, to alternately receive and expel blood. One way valves at the inlet and outlet ports of the chambers ensured one way flow therethrough.

So-called "pulsatile pumps" may include one or a pair of driven plates for alternately squeezing and expanding flexible chambers. The flexible chambers typically comprise biocompatible segmented polyurethane bags or sacs. The blood sac and drive mechanism are mounted inside a compact housing that is typically implanted in the patient's abdomen. A controller, backup battery, and main battery pack are electrically connected to the drive mechanism. Even the most basic drive mechanisms of the prior art are relatively complex and expensive, and typically incorporate some type of mechanical cam, linkage, or bearing arrangement subject to wear.

Because of the varying volume of the blood sac within the rigid encapsulation housing of pulsatile pumps, accommodation must be made for the air displaced thereby. Some devices utilize a percutaneous tube vented to the atmosphere, which is a simpler approach but involves skin penetration. Another possible approach for fully-implantable VAD systems is to use a volume compensator. This is a flexible chamber, implanted in the thoracic cavity adjacent to the lungs and communicating with the air space within the housing and outside the blood sac via an interconnecting tube. As the blood sac expands with incoming blood, air is displaced from the housing to the volume compensator. Conversely, expulsion of blood from the blood sac creates a negative pressure within the housing and pulls air from the volume compensator. While eliminating the infection risk due to the skin penetration of the percutaneous vented tube, the volume compensator poses certain challenges: increased system complexity, an additional implanted component and potential site of infection, maintaining long-term compliance of the implanted volume compensator sac, problems associated with gas diffusion in or out of the enclosed volume, and problems associated with changes in ambient pressure, such as experienced during a plane flight.

One example of an electric pulsatile blood pump is the Novacor N100 Left Ventricular Assist System (World Heart Inc., Oakland, Calif.). This system contains a single polyurethane blood sac with a nominal stroke volume of 70 ml that is compressed by dual symmetrically opposed pusher plates in synchronization with the natural left ventricle contraction. The pusher plates are actuated by a spring-decoupled solenoid energy converter. The blood pump and energy converter are contained within a housing that is implanted in the patient's abdomen. The N100 employs a percutaneous vent tube that also carries power and control wires.

An example of an electric pulsatile blood pump not requiring external venting is disclosed in U.S. Patent No. 6,264,601 ("the '601 patent"). The system of the '601 patent has two pumping chambers formed from two flexible sacs separated by a pusher plate, with the sacs and pusher plate contained within one housing. A electromagnetic drive system acts on an iron armature surrounded by a cylindrically symmetric permanent magnet within the pusher plate to alternatively pump blood through the two sacs by compressing one sac and then the other against the housing. The electromagnetic drive is also referred to herein as the direct magnetic drive (DMD). Since each sac contains only fluid that is alternately received and discharged as the pusher plate reciprocates, the total volume of the pump remains constant during pumping and no venting or volume compensator is required. The input and output of each sac includes a one-way valve, providing unidirectional flow that pumps the fluid in a preferred direction. The most efficient use of the electromagnetic drive system is achieved when the power and energy required in each pump stroke is approximately equal.

The '601 patent describes several alternative arrangements for using a blood pump, including a left or right VAD that couples the input and output flows from each chamber in either parallel or series, and a BVAD that separately uses two separate VADs to assist the left and right ventricle. One embodiment described in the '601 patent is a series-displacement pump, in which a first chamber receives a fluid for pumping, and provides that fluid to the input of a second chamber for further pumping ("the '601 series-displacement pump"). In operation, the '601 series displacement pump alternates between a pump stroke and a transfer stroke. When used as a VAD, the pump stroke pumps blood from the second chamber into the aorta while blood is drawn from the ventricle into the first chamber. In the transfer stroke, blood from first chamber is transferred to the second chamber. The fluid connection between the chambers is an external transfer conduit that connects the output of the first sac to the input of the second sac.

The '601 series-displacement pump has several advantages over other prior art pumps including, but not limited to, the ability to provide pulsatile flow, the use of fewer blood conduits and valves, and reduced size. However, the electromagnetic drive system of the '601 patent is optimized for bi-directional use, while the power and transfer strokes of the '601 series-displacement pump each have different power and energy characteristics. While the pump of the '601 patent is capable of operating as a series-displacement pump, there are energy losses that result from not having the drive and pump matched for series operation. Also, in general, the pump of the '601 patent includes a permanent magnet to drive the pusher plates that has a radially symmetric design that is expensive and difficult to manufacture.

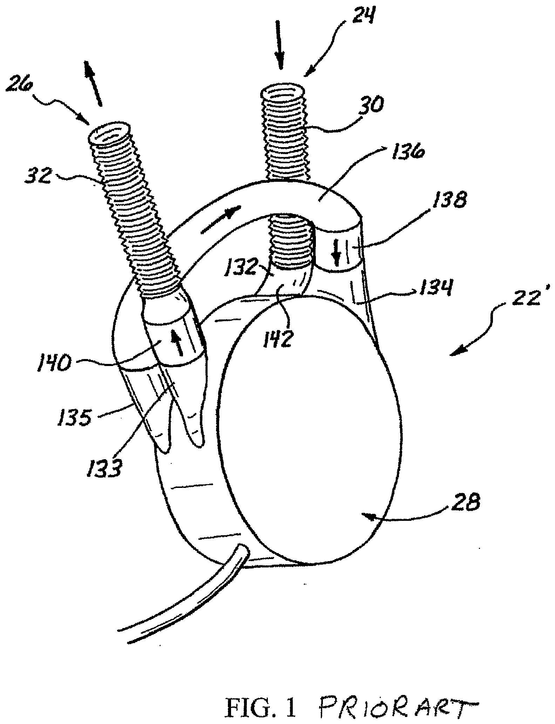

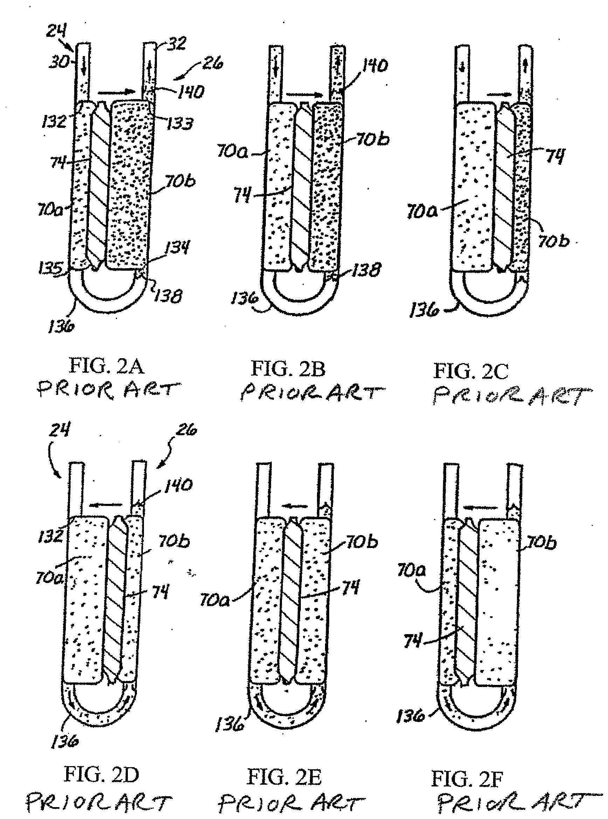

A Pump/Drive Unit (PDU) is one of the configurations of the DMD described in the '601 patent as the "Series-displacement VAD". As described in the '601 patent, a pump 28 is configured in a ventricular assist system 22' shown in FIGs. 1 and 2A-2F in which the chambers 70 are connected in series. A flexible inlet segment 30 of the inlet conduit 24 connects to, for example, the left ventricle of the heart, not shown, and the flexible outlet segment 32 of the outlet conduit 26 connects to, for example, the aorta, not shown. In this embodiment, the flexible inlet segment 30 is only connected to the inlet port 132 of one of the chambers, such as the left chamber 70a, and the flexible outlet segment 32 is only connected to the outlet port 133 of the other chamber, such as the right chamber 70b.

According to the embodiment shown in FIG. 1, a transfer conduit 136 is connected between the outlet port 135 of the chamber connected to the flexible inlet segment 30 (the left chamber 70a) and to the inlet port 134 of the chamber connected to the flexible outlet segment 32 (the right chamber 70b). In addition, a pair of valves are provided, including an inlet valve 138 disposed at the inlet port 134 of the chamber connected to the outlet conduit 26 and an outlet valve 140 disposed at the outlet port 133 of the chamber connected to the outlet conduit 26.

In accordance with the series flow blood pump 28 exemplified in FIG. 1, blood from the left ventricle is initially pumped to the left chamber 70a in the inlet conduit 24. Coils, not visible in the views of FIGs. 1-2F, are activated to move the plate 74 to the right as shown by the arrow in FIG. 2A, thereby ejecting blood received within the right chamber 70b through the outlet port 133 and the outlet valve 140 and into the flexible outlet segment 32 for delivery to the aorta. During this ejection stroke of the plate 74, the inlet valve 138 prevents blood from entering the transfer conduit 136. In addition, the left chamber 70a is expanded, thereby drawing oxygenated blood through the inlet conduit 24 from the left ventricle (not shown) into the left chamber as shown in FIG. 2B. At the end of the ejection stroke as shown in FIG. 2C with the plate 74 positioned to the right, the left chamber 70a is filled with oxygenated blood from the left ventricle, and the right chamber 70b is compressed to a minimum volume.

The coils are then activated to move the plate 74 to the left as shown by the arrows in FIGS. 2D and 2E, thereby drawing blood from the left chamber 70a into the right chamber 70b via the transfer conduit 136. The outlet valve 140 prevents blood in the aorta or the outlet conduit 26 from being drawing back into the right chamber 70b. In addition to left ventricular pressure, the low pressure within the right chamber 70b caused by the expansion of the chamber ensures that blood within the left chamber 70a enters the right chamber 70b and is not ejected back into the inlet conduit 24. If desired, an additional valve may be disposed at the inlet port 132 of the left chamber 70a to also prevent blood from entering the inlet conduit 24. At the end of the transfer stroke as shown in FIG. 2F with the plate 74 positioned to the left, the right chamber 70b is filled with oxygenated blood from the left ventricle, and the left chamber 70a is compressed to a minimum volume. The ejection stroke illustrated in FIGS. 2A-2C and the transfer stroke illustrated in FIGS. 2D-2F may be repeated in accordance with the exemplary methodology of the invention described above. Control of the system 22' may be based on the sensed input pressure. An advantage of the ventricular assist system 22' is the reduction of the number of valves needed, from four to two. This in turn reduces the cost of the device.

Thus, the configuration illustrated in FIGs. 1 - 2F transfers blood between the two pumping chambers, and ejects into the aorta only from the second chamber. The configuration therefore requires only two valves, rather than four, and avoids the complication of bifurcated conduits. The pumping chamber shown in FIG. 1 ejects into the aorta through the outflow valve, while the "pre-chamber" sac, at the left in FIG. 1, serves as the displacement chamber and communicates with the left ventricle via a non-valved conduit. Since this VAD functions with no vent or compliance device, its total volume is constant so the inflow and outflow are always equal.

The coils of the VAD pump shown in FIG. 1 are housed within a frame and disposed in a spaced relationship to generate a coil flux through a pair of poles. The pusher plate including an armature and a magnet is disposed within the frame such that the armature is between the poles and the magnet is between the coils. As described in the '601 patent, gaps are defined between the armature and each of the poles and the coil flux displaces the armature across the gap. The coil flux follows a donut-shaped path including the frame, one of the poles, one of the gaps, the armature, the other the gap, and the other the pole, and no coil flux passes through the magnet. The magnet generates a bias flux that offsets a portion of the coil flux around the frame. A spring is optionally provided to counteract the unstable force balance developed by the bias magnet.

A large central pole is associated with each coil of the pair of coils for the device in FIGs. 1 and 2 and its armature's permanent magnet (PM) flux is returned via lateral gaps. A drawback of the device described the '601 patent is that the centered armature is at a lateral unstable equilibrium point. The pump sac flexures of the device, in practice, proved marginally stiff enough to stop the PM from pulling the armature to the side. What is needed is an electromagnetic drive that eliminates this lateral instability.

What is also needed is a an electromagnetic drive having a geometry that eliminates the lateral magnetic gaps of the known design variants since the gaps require magnetization, and therefore increased PM size, but contributes nothing to useful force. What is also needed is elimination of the large stator return bars or cylinder which are a major part of the size of the electromagnetic drive. What is needed is a simpler, more efficient electromagnetic drive of reduced size, weight and complexity for use in a ventricular assist device to facilitate use thereof. An electromagnetic drive is known from DE 3804768.

## SUMMARY OF THE INVENTION

The present invention provides an electromagnetic drive for use in a ventricular assist device.

The invention is defined by independent claim 1. The present invention as claimed provides an electromagnetic drive comprising a pair of U-shaped cores, each having a center section and two legs; one or more coils, each coil wound around a selected one of the U-shaped cores that, when electrically energized, generate a magnetic flux and define one or more pairs of magnetic poles; and an armature between the U-shaped cores, having two non-permanent-magnetic ends and a permanent magnet therebetween, the magnet for generating a magnet force on the armature resulting from the attraction of the magnet to the U-shaped cores when the coils are not electrically energized, wherein the energized one or more coils generate a coil force on the armature that is approximately independent of the position of the armature and that varies according to the degree of energization of the coils.

The electromagnetic drive of the present invention has the advantage of reducing the lateral instability and size of the drive and corresponding ventricular assist device. The drive has the further advantage of increasing magnetic efficiency and linearity as compared to known drives.

These and other embodiments, features, aspects, and advantages of the invention will become better understood with regard to the following description, appended claims, and accompanying drawings.

## BRIEF DESCRIPTION OF THE DRAWINGS

The foregoing aspects and the attendant advantages of the present invention will become more readily appreciated by reference to the following detailed description, when taken in conjunction with the accompanying drawings, wherein:

FIG. 1 is perspective view of a blood pump shown in U.S. Patent No. 6,264,601 in which variable-volume chambers operate in series;
FIGS. 2A to 2F are schematic views of the blood pump of FIG. 1, illustrating the variable-volume chambers of the pump operating in series;
FIG. 3 is a side view of an C-core electromagnetic drive according to an embodiment of the present invention;
FIG. 4 is a schematic top view of an C-core electromagnetic drive according to an embodiment of the present invention;
FIG. 5 is a field plot 300 of the central section at midstroke, showing gap field uniformity and leakage flux containment for the C-Core electromagnetic drive shown in FIGs. 3 and 4;
FIG. 6 illustrates how, for an integrated drive having an armature, the sacs of the VAD lie within the working magnetic gaps;

FIG. 7 is a schematic according to an embodiment of the present invention showing sacs and an axially separated electromagnetic drive, identified as a "DMD unit";

FIG. 8 is a schematic sectional view of a U-core electromagnetic drive for coaxial placement according to a preferred embodiment of the present invention;

FIG. 9 is a schematic top view of the U-core electromagnetic drive in FIG. 8;

FIG. 10 is a finite element analysis (FEA) gap field map of the U-core electromagnetic drive in FIG. 8, showing less fringing flux than for the C-core electromagnetic drive in FIG. 3, and

FIG. 11 is a graph illustrating the force for exemplary U-core electromagnetic drive, e.g., FIG. 8, versus the force for the exemplary C-core electromagnetic drive, e.g., FIG. 3, by FEA for the same pole area and stroke.

Reference symbols are used in the Figures to indicate certain components, aspects or features shown therein, with reference symbols common to more than one Figure indicating like components, aspects or features shown therein.

## DETAILED DESCRIPTION OF THE INVENTION

The large central pole associated with each coil of the pair of coils for the electromagnetic drive in the system shown in FIGs. 1-2F and the armature's permanent magnet (PM) flux is supplanted by the geometry topologies of the electromagnetic drive embodiments according to the present invention. One embodiment of the electromagnetic drive of the present invention is referred to herein as the "C-core" electromagnetic drive topology, "C-core drive" or just "C-core". The design approach of a "C-Core" drive topology sought to find the inherent electromagnetic drive performance envelope as defined only by functional targets including pressure, stroke volume, etc., mostly unconstrained by pump considerations. One requirement that was retained is that the electromagnetic drive is not wider than the pump. This requirement allows functional separation between the stator, the pole structure, and, partially, the field magnet (PM). In this design approach, the number of interactive parameters for each part is reduced to a manageable level. Reliance on assumptions that appeared plausible, but were determined in practice to be incorrect in many instances, is markedly reduced in this design approach.

Design optimization proceeded from a model analysis starting with the electromagnetic drive force equation as modified for the C-core drive:

$$F_{MAG} = \Phi^2 \, x/2\mu_O gA(1+\Psi g) + NI\Phi/g, \tag{1}$$

in SI units, where $\Phi$ is said permanent magnet's flux, x is said armature's deflection from center, $\mu_O \equiv 4\pi E\text{-}7$, g is the mid-stroke gap, A is the pole area, NI the ampere-turns per said coil and $\Psi$ is a gap spreading factor used to approximate the effect of leakage flux. Equation (1) is a simplified equation for illustrative purposes, it ignores several often significant factors including nonlinearity produced by stator core saturation, especially at end of stroke; variation of flux $\Phi$ with position which will be significant if the PM is shortened to save weight; and leakage flux which is nonlinear in the gap and will therefore not cancel in the NI term as shown.

With regard to core saturation, an electromagnetic drive is more vulnerable to saturation than a single-path magnetic structure like the solenoid used in known designs such as the Novacor N100. A reason for this increased vulnerability is that the "other" stator core is always nearby to attract any flux that spills from a saturated stator core. Unlike normal fringing fields, this spill directly subtracts from the intended electromagnetic drive force. The stator cores must be sized to accommodate the sum of PM and coil fluxes if the end-of-stroke residual gap is near zero. This requirement is costly in size and weight, an incentive to cut the saturation margin to a minimum. Saturation then readily occurs when coil current is raised above design level.

Equation (1) approximates the PM flux $\Phi$ at its midstroke value. The nonlinearity of the leakage flux in the gap has been curbed in known devices by making the PM much longer than needed to excite the gap, i.e., loading it lightly so permeance changes do not affect the flux. A drawback of that method in known devices of curbing nonlinearity is the unproductive size and weight due to the much longer PM.

The PM force represented by the first term in equation (1) is compensated by a spring array whose rate k = $\Phi^2/2\mu_O gA$ and whose neutral point is at x = 0. If the neutral point is offset by the distance d from the center, then the spring force is

$$F_S = -k(x-d),$$

and the differential pusher plate force is

$$F_S + F_{MAG} = \ kd + NI\Phi/g.$$

Simply offsetting the spring therefore produces a constant eject assist force. Normally, a constant-force spring, such as needed for the eject assist, is difficult to realize. The electromagnetic drive according to an embodiment of the present invention provides this feature merely by adjustment.

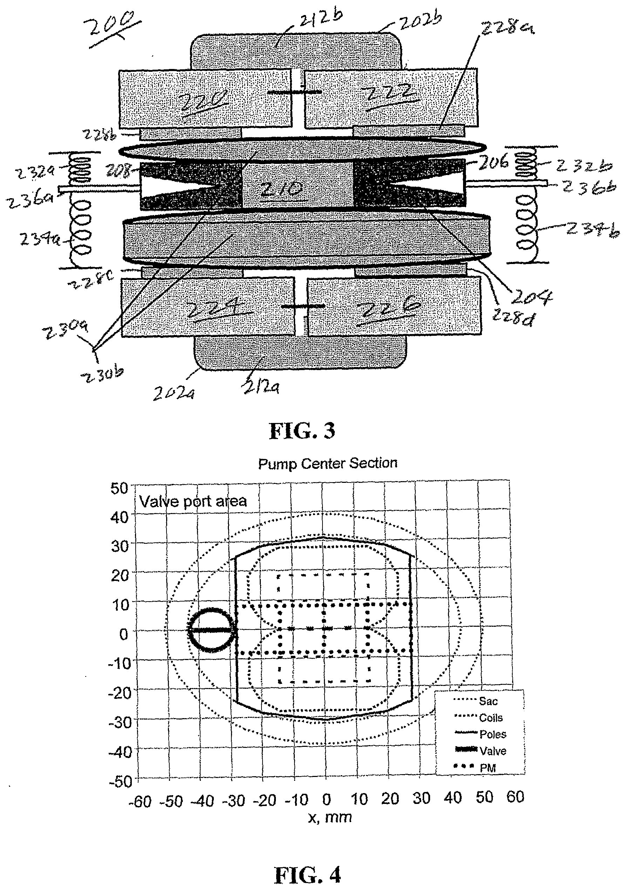

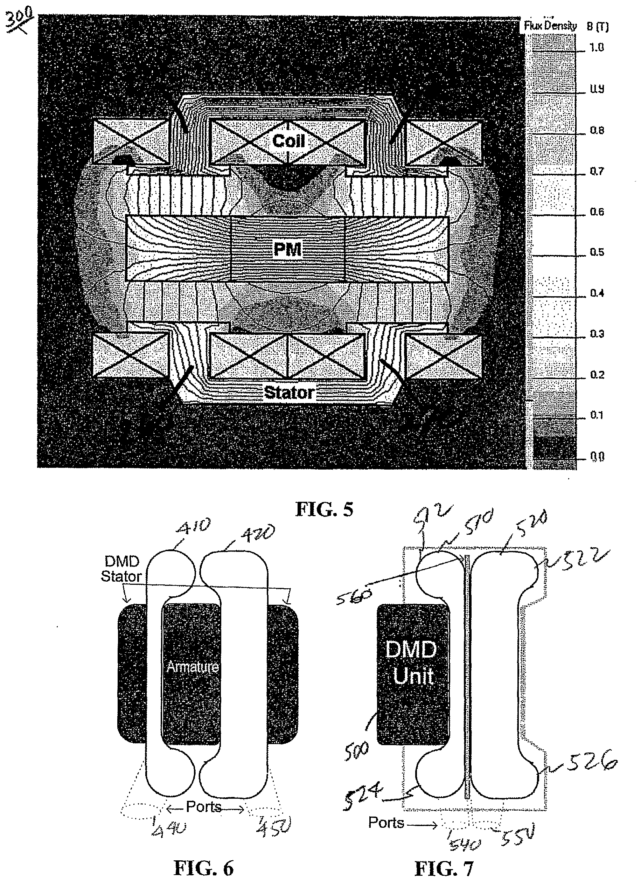

FIG. 3 is a side view of a C-core electromagnetic drive 200 according to an embodiment of the present invention. FIG. 4 is a schematic top view of a C-core electromagnetic drive 200 according to an embodiment of the present invention. The electromagnetic drive of the present invention is preferably used in a VAD. FIG. 4 also shows a valve of the VAD with respect to the drive elements. The C-core electromagnetic drive 200 includes a pair of stator cores 202a, 202b having respective center sections 212a, 212b as identified in FIG. 3. The cores 202a, 202b are preferably U-shaped such that each core also has two legs. Drive 200 has two pairs of adjacent coils with coils 220 222, wound respectively around the two legs (shown in FIG. 5 as legs 240, 250) of core 212b and coils 224, 226 wound respectively around the two legs (shown as legs 260, 270 for the other core, identified as "stator", in FIG. 5) of core 212a. According to an alternate embodiment, the electromagnetic drive includes one pair of coils with each of the coils wound around a respective leg of one of the U-shaped cores, and the other U-shaped core is coil-less.

The electromagnetic drive 200 includes an armature 204 having ends 206, 208 and a permanent magnet 210 therebetween. The coils generate a flux component that passes transversely through the armature 204. Poles shoes 228a, 228b are coupled to respective ends of core 202b. Pole shoes 228c and 228d are coupled to respective ends of core 202a. FIG. 3 shows sacs 230a and 230b of the pump of the VAD located on opposing sides of armature 204. That is, the sacs 230a, 230b are part of the PDU combination pump/drive of the VAD, but are not part of the electromagnetic drive according to the present invention.

One or more springs are alternatively included as a storage element. In the embodiment shown in FIG. 3, a spring 232a coupled via radial arm 236a to a spring 234a on one side of the drive, and springs 232b is coupled via radial arm 236b to a spring 234b on the other side. The springs 232a, 234a and 232b, 234b are in pairs and are preloaded. Consequently, the springs are in compression throughout the ejection stroke which minimizes the fatigue effect of reciprocating load, and simplifies spring retention. The ejection or eject stroke is also referred to as the pump stroke herein.

The geometry of the electromagnetic drive of the present invention is preferably optimized through use of a model combining electromagnetic drive performance targets, an empirical pump hydraulic ΔP model, and pump parameters into a force vs. stroke relation. The force equation (1) supported by magnetostatic relations for the PM and coil magnetic circuits, is backsolved to obtain required coil ampere-turns (NI). These determinations and the specified dissipation determine coil geometry and finally electromagnetic drive volume, as a function of electromagnetic drive shape parameters. The shape giving the smallest size is then found by numerical computation and charting over ranges of drive shape parameters, in a spreadsheet, for example. Finally, the assumptions in this modeling process, particularly fringing fields (as characterized in the "gap spreading" factor Ψ) and saturation level of the core, are tested by script-driven parametric finite element analysis (FEA). Assumed constants are adjusted as necessary and the process iterated until closure between FEA and the closed-form model is reached.

FIG. 5 is a field plot 300 of the central section at midstroke, showing gap field uniformity and leakage flux containment for the C-Core electromagnetic drive embodiment shown in FIGs. 3 and 4. FEA predicted low leakage flux because the device's inherent symmetry results in the fringing field cancellation shown in FIG. 5, and thus good magnetic efficiency and linearity.

The resulting integrated electromagnetic drive embodiment is elegant yet simple. As seen in FIGs. 3, the armature 204 occupies the central space surrounded by the sacs 230a, 230b and the stator cores 212a, 212b. Alternatively, the sacs 230a, 230b include sac flexures for stabilization (not shown in FIG. 3). Radial arms 236a, 236b, schematically shown in FIG. 3, preferably connect to the array of springs around the drive perimeter. The stator core's diameter, to match that of the armature 204, is considerably less than the diameter of the pump, to allow for the flex region. This stator diameter leads to an anatomically desirable "oyster" shape for the pump and drive combination, i.e., the PDU, of a VAD.

The pole shoes, shown as 228a, 228b, 288c, 228d in FIG. 3, coupled to respective legs of the stator core are included for minimizing coil resistance, for reducing saturation of the respective core. A large pole area (A in Equation 1) is desirable because it increases overall gap permeance, allowing a smaller PM to develop the desired bias flux. A large pole area also reduces gap spreading factor Ψ, improving linearity. In addition, a large pole lowers the PM negative "spring constant" and therefore the physical spring needed to compensate it, without affecting coil current force. This reduces structural overhead as well as the dimensional precision required by very stiff springs. It follows from a relatively large pole area that the gap field, BGAP, will be far below core saturation. The coils can afford to surround only the smallest section of core iron necessary to carry the peak flux. Thus, the pole shoes will always be much wider than the core legs, as shown in the example in FIG. 3. Some flux passes directly from the cores 212a, 212b into the gap, but the rest must pass transversely into the respective shoes 228a, 228b, 228c, 228d. This imposes a minimum shoe thickness where they join the legs of the cores 212a, 212b. Ideally, the shoes 228a, 228b, 288c, 228d taper to zero thickness at their free edges. The stator core 212a, 212b and corresponding coils 220, 222, 224, 226 may intersect the respective shoes 228a, 228b, 228c, 228d anywhere thereon. In the range of interest, the shoes 228a, 228b, 228c, 228d are preferably big enough so as to not limit the shape of near-optimum stator cores 212a, 212b so the stator cores 212a, 212b can be designed independently for minimum size.

For an integrated electromagnetic drive according to an embodiment shown in FIG. 3, the sacs 230a, 230b of the pump of the VAD lie within the working magnetic gaps. FIG. 6 illustrates how, for an integrated drive having an armature 430 as shown, the sacs 410, 420 of the VAD lie within the working magnetic gaps. The minimum blood layer across each "empty" sac is at least 3-4 mm. This "empty" thickness degrades electromagnetic drive magnetic efficacy, since it represents a large volume which must be magnetically energized with coil power, but from which mechanical work cannot be harvested. Minimizing that effect favors a long pump stroke, of which the "empty" thickness is a lesser fraction. In addition to making the PDU too thick, that expedient brings its own problems. Flex region width as defined by sac stress analysis rises rapidly with stroke, leaving the pumps with little flat area available for the core poles of the electromagnetic drive. This lack of flat area leads to an unfavorable ratio of pole width to gap, and consequent high fringing flux and poor magnetic performance. It also increases PDU volume by adding flex region overhead. A tightly coupled electromagnetic drive with much lower fringing flux is realized by separating it from the pumps.

FIG. 7 is a schematic according to an embodiment showing sacs 510, 520 and an axially separated electromagnetic drive 500, identified as a "DMD unit". The drive 500 is preferably coupled to an actuator such as pusher plates. To make this electromagnetic drive as flat as possible, a single coil wound around a long, flat stator core is preferably substituted for each pair of coils and corresponding core of the C-core electromagnetic drive in FIG. 3. The electromagnetic drive according to a preferred embodiment of the present invention having a single coil wound around a center section of a long, flat stator core is also referred to herein as a U-core electromagnetic drive or U-core. The U-core electromagnetic drive fits especially well with the axially separated pump arrangement shown in FIG. 7. FIG. 7 shows a septum 560 between sacs 510 and 520 that is no longer part of the electromagnetic drive, its function is only mechanical. According to one embodiment, the septum 560 is fixed and the sac flexures 512, 522, 524, 526 may face outward as shown with the VAD's ports 540, 550 relocated adjacent to the septum 560, as opposed to the separation of the ports 440, 450 shown in FIG. 6. According to an alternative embodiment, a moving septum is included and the sec flexures may face inward, as shown in FIG. 6. In the arrangement shown in FIG. 7, the armature (details not shown in FIG. 7) of electromagnetic drive 500 is not constrained in shape by its interface with the actuator's pusher plates and the sac flex regions. Since the gap fields are approximately uniform, it follows that farther away from the PM, less armature iron is needed to distribute the near-constant PM flux component, which suggests a tapered armature.

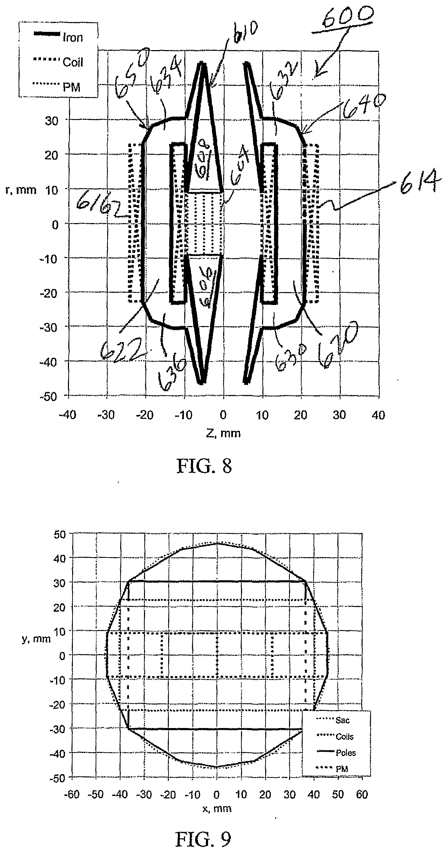

FIG. 8 is a schematic sectional view of a U-core electromagnetic drive 600 according to a preferred embodiment of the present invention. The U-core electromagnetic drive 600 has a tapered armature 610 for coaxial placement. Armature 610 has two tapered ends 606, 608 and a permanent magnet (PM) 604 therebetween. As shown schematically in Fig. 8, drive 600 has two coils 614, 616 each wound around a respective center section 620, 622b of a respective U-shaped core 640, 650. U-shaped core 640 has a pair of legs 630, 632. U-shaped core 650 has a pair of legs 634 and 636. According to an alternative U-core electromagnetic drive embodiment, the electromagnetic drive has only one coil and that coil is wrapped around a selected one of the U-shaped core's center sections 620, 622 and the other U-shaped core has no coil wrapped therearound, i.e., the other U-shaped core is coil-less.

For the electromagnetic drive according to the present invention, each coil wound around a U-shaped core, when electrically energized, generates a magnetic flux and define one or more pairs of magnetic poles each having a polar axis. Preferably, one or more pairs of gaps are defined between the armature and each one of said one or more pairs of magnetic poles. The drive preferably has stator poles located at ends of the legs of said U-shaped core.

FIG. 9 is a schematic top view 600a of the U-core electromagnetic drive 600 of FIG. 8. The coil-generated flux component simply passes transversely through the armature 610, so no minimum thickness is required for the armature 610. Matching the stator pole shape to the tapered armature 610 results in a volumetrically efficient nested electromagnetic drive 600, as shown in FIGs. 8 and 9.

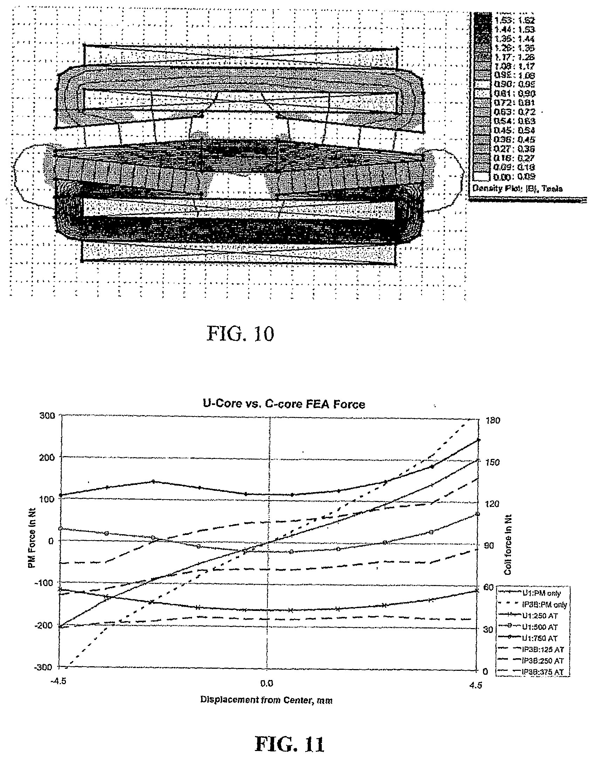

A further advantage of the U-core electromagnetic drive 600 shown in FIGs. 8 and 9 is that its narrow pole edges minimize fringing permeance since less magnetized surface is exposed to regions other than the working gaps. Leakage flux in the U-core electromagnetic drive 600 is accordingly substantially less than in the equivalent C-core electromagnetic drive, e.g. shown in FIGs. 3 and 4. FIG. 10 shows an exemplary FEA gap field map for the U-core electromagnetic drive 600 showing less fringing flux than for the C-core electromagnetic drive, e.g., C-core electromagnetic drive 200 shown in FIGs. 3 having a field map plotted as shown in FIG. 5. The reduced fringing flux for the U-core electromagnetic drive according to the preferred embodiment means that there is less tendency of the stator core to saturate at end-stroke. As a result there is better linearity and higher specific output for the U-core electromagnetic drive and its geometry shown in FIGs. 8 and 9.

FIG. 11 is a graph illustrating force for the U-core electromagnetic drive, e.g., FIG. 8, as compared to the force for a C-core electromagnetic drive, e.g., FIG. 3, by FEA for same pole area and stroke. U1 designates a U-core electromagnetic drive, and IP3B designates a C-core electromagnetic drive in the key in FIG. 11. The U-core electromagnetic drive, represented by the solid line waveforms, has only 2 coils in the preferred embodiment shown in FIG. 8; thus each has twice the amp-turns of each of the four coils in the C-core electromagnetic drive in FIG. 3. FIG. 11 also illustrates the U-core electromagnetic drive's improved output, less saturation loss, indicated by the force droop at left, and better linearity.

The spring arrangement shown in FIG. 3 is alternately also used in conjunction with a U-core electromagnetic drive. Accordingly, the electromagnetic drive preferably includes a frame and one or more springs positioned between the frame and the armature so as to exert a spring force on the armature, wherein the sum of the spring force and the magnetic force is approximately zero and is approximately independent of the position of the armature along the polar axis. Alternatively, the sum of the spring force and the magnetic force is a net bias force that is approximately independent of the position of the armature along the polar axis and biases the armature towards one of the pair of poles, such that a constant eject assist force is obtained by simply offsetting the springs. The armature is preferably held in a retracted position toward a first one of the U-shaped cores and against the eject assist force of the springs solely by the magnet force with substantially no energization of the one or more coils by having a gap between the armature and the U-shaped cores become substantially small in the retracted position. The magnet force preferably overcomes the spring force as the armature moves toward the first one of the U-shaped cores such that the armature is magnetically latched in the retracted position.

The drive according to the present invention is for use in a ventricular assist device having one or more sacs.

The invention is defined by the appended claims.

## Claims

1. An electromagnetic drive (200) comprising:

   a pair of U-shaped cores (202), each having a center section (212)and two legs;
   one or more coils (220, 222, 224, 226), each coil wound around a selected one of said U -shaped cores (202) that, when electrically energized, generate a magnetic flux and define one or more pairs of magnetic poles;
   an armature (204) between said U-shaped cores (202), having two non-permanent-magnetic ends (206, 208) and a permanent magnet (210) there between, said magnet (210) for generating a magnet force on said armature (204) resulting from the attraction of said magnet (210) to said U-shaped cores (202) when said coils (220, 222, 224, 226) are not electrically energized; wherein said energized one or more coils generate a coil force on said armature that is approximately independent of the position of said armature and that varies according to the degree of energization of said coils; and **characterized by**
   two pairs of pole shoes (228), each pole shoe (228) coupled to an end of a leg of the U-shaped cores (202), whereas the pole shoes (228) are wider than the legs of the U-shaped cores (202).

2. The drive (200) of Claim **1,** wherein said drive has two said coils (220, 222, 224, 226) each wound around a respective U-shaped core's center section (212).

3. The drive (200) of Claim **1,** wherein said drive has a single one of said coils (220, 222, 224, 226), said coil (220, 222, 224, 226) wrapped around said selected one of said U-shaped core's center sections (212), wherein the other of said U-shaped cores (202) is coil-less.

4. The drive (200) of Claim **1,** wherein said drive has two pairs of said coils (220, 222, 224, 226) with each of said coils (220, 222, 224, 226) wound around a respective leg of said U-shaped cores (202) and each said pair having two adjacent coils (220, 222, 224, 226).

5. The drive (200) of Claim **1,** wherein said drive has one pair of said coils (220, 222, 224, 226) with each of said coils (220, 222, 224, 226) wound around a respective leg of one of said U-shaped cores (202), wherein the other of said U-shaped cores (202) is coil-less.

6. The drive (200) of Claim **1,** wherein said one or more coils (220, 222, 224, 226) generates a flux component that passes transversely through said armature (204).

**7.** The drive (200) of Claim **1,** wherein said non-permanent-magnetic ends (206, 208) of said armature (204) are tapered so as to further reduce the size of said drive.

**8.** The drive (200) of Claim **1,** wherein one or more pairs of gaps are defined between said armature (204) and each one of said one or more pairs of magnetic poles, and said drive having narrow pole edges so as to reduce fringing permeance of said drive.

**9.** The drive (200) of Claim **1,** wherein said drive has stator poles located at ends of said legs of said U -shaped core (202).

**10.** The drive (200) of Claim **1,** further comprising a frame and one or more springs (232) positioned between said frame and said armature (204) so as to exert a spring force on said armature.

**11.** The drive (200) of Claim 10, wherein the sum of said spring force and said magnetic force is approximately zero and is approximately independent of the position of said armature (204).

**12.** The drive (200) of Claim 10, wherein the sum of said spring force and said magnetic force is a net bias force that is approximately independent of the position of said armature (204) and biases said armature (204) towards one of said pair of poles, such that a constant eject assist force is obtained by offsetting said springs (232).

**13.** The drive (200) of Claim 10, wherein said armature (204) is held in a retracted position toward a first one of said U-shaped cores (202) and against said eject assist force of said springs (232) solely by said magnet force with substantially no energization of said one or more coils (220, 222, 224, 226) by having a gap between said armature (204) and said U -shaped cores (202) become substantially small in said retracted position.

**14.** The drive (200) of Claim 13, wherein said magnet force overcomes said spring force as said armature (204) moves toward said first one of said U -shaped cores (202) such that said armature (204) is magnetically latched in said retracted position.

**15.** The drive (200) of Claim 1, wherein the thickness of the pole shoes (228) taper to zero thickness at their free edges.

**16.** A ventricular assist device having one or two sacs (230) and a drive (200) according to Claim **1.**

**17.** The drive (200) of Claim 1, wherein the geometry of said drive is optimized based on a model that combines performance targets for said drive, an empirical pump hydraulic pressure model, and pump parameters into a force versus stroke relation.

**Patentansprüche**

**1.** Elektromagnetischer Antrieb (200), umfassend:

ein Paar U-förmiger Kerne (202), jeweils mit einem zentralen Abschnitt (212) und zwei Schenkeln;
eine oder mehrere Spulen (220, 222, 224, 226), wobei jede Spule um einen ausgewählten der U-förmigen Kerne (202) gewickelt ist, die, wenn sie elektrisch erregt werden, einen Magnetstrom erzeugen und einen oder mehrere Paare magnetischer Pole definieren;
einen Anker (204) zwischen den U-förmigen Kernen (202) mit zwei nicht-permanenten magnetischen Enden (206, 208) und einem Permanentmagneten (210) dazwischen, der Magnet (210) zum Erzeugen einer Magnetkraft auf den Anker (204), die aus der Anziehung des Magneten (210) auf die U-förmigen Kerne (202) resultiert, wenn die Spulen (220, 222, 224, 226) nicht elektrisch erregt sind; wobei die elektrisch erregte(n) eine oder mehreren Spule(n) eine Spulenkraft auf den Anker erzeugt/erzeugen, die ungefähr unabhängig von der Position des Ankers ist und die entsprechend dem Grad der Erregung der Spulen variiert; und **gekennzeichnet durch** zwei Paare von Polschuhen (228), wobei jeder Polschuh (228) mit einem Ende eines Schenkels der U-förmigen Kerne (202) gekoppelt ist, während die Polschuhe (228) breiter als die Schenkel der U-förmigen Kerne (202) sind.

**2.** Antrieb (200) nach Anspruch 1, wobei der Antrieb zwei Spulen (220, 222, 224, 226) aufweist, die jeweils um einen jeweiligen zentralen Abschnitt (212) eines U-förmigen Kerns gewickelt sind.

**3.** Antrieb (200) nach Anspruch 1, wobei der Antrieb eine einzige der Spulen (220, 222, 224, 226) aufweist, wobei die Spule (220, 222, 224, 226) um den ausgewählten der zentralen Abschnitte (212) der U-förmigen Kerne gewickelt ist, wobei der andere der U-förmigen Kerne (202) spulenlos ist.

4. Antrieb (200) nach Anspruch 1, wobei der Antrieb zwei Paare der Spulen (220, 222, 224, 226) aufweist, wobei jede der Spulen (220, 222, 224, 226) um einen jeweiligen Schenkel der U-förmigen Kerne (202) gewickelt ist und jedes Paar zwei angrenzende Spulen (220, 222, 224, 226) aufweist.

5. Antrieb (200) nach Anspruch 1, wobei der Antrieb ein Paar der Spulen (220, 222, 224, 226) aufweist, wobei jede der Spulen (220, 222,224, 226) um einen jeweiligen Schenkel eines der U-förmigen Kerne (202) gewickelt ist, wobei der andere der U-förmigen Kerne (202) spulenlos ist.

6. Antrieb (200) nach Anspruch 1, wobei eine oder mehrere Spulen (220, 222, 224, 226) eine Strömungskomponente erzeugt, die den Anker (204) quer durchläuft.

7. Antrieb (200) nach Anspruch 1, wobei sich die nicht-permanenten magnetischen Enden (206, 208) des Ankers (204) verjüngen, um die Größe des Antriebs weiter zu reduzieren.

8. Antrieb (200) nach Anspruch 1, wobei eines oder mehrere Paare von Spalten zwischen dem Anker (204) und jedem des einen oder der mehreren Paare von magnetischen Polen definiert sind, und der Antrieb schmale Polkanten aufweist, um eine Randdurchlässigkeit des Antriebs zu verringern.

9. Antrieb (200) nach Anspruch 1, wobei der Antrieb Statorpole aufweist, die an den Enden der Schenkel des U-förmigen Kerns (202) positioniert sind.

10. Antrieb (200) nach Anspruch 1, ferner umfassend einen Rahmen und ein oder mehrere Federn (232), die zwischen dem Rahmen und dem Anker (204) positioniert sind, um eine Federkraft auf dem Anker auszuüben.

11. Antrieb (200) nach Anspruch 10, wobei die Summe der Federkraft und der magnetischen Kraft ungefähr Null ist und ungefähr unabhängig von der Position des Ankers (204) ist.

12. Antrieb (200) nach Anspruch 10, wobei die Summe der Federkraft und der magnetischen Kraft eine Netto-Vorspannungskraft ist, die ungefähr unabhängig von der Position des Ankers (204) ist und den Anker (204) zu einem der Paare der Pole vorspannt, so dass durch Versetzen der Federn (232) eine konstante Auswurfhilfskraft erhalten wird.

13. Antrieb (200) nach Anspruch 10, wobei der Anker (204) in einer eingezogenen Position zu einem ersten der U-förmigen Kerne (202) und gegen die Auswurfhilfskraft der Federn (232) nur durch die Magnetkraft mit im Wesentlichen keiner Erregung der einen oder der mehreren Spule(n) (220, 222, 224, 226) gehalten wird, indem ein Spalt zwischen dem Anker (204) und den U-förmigen Kernen (202) in der eingezogenen Position im Wesentlichen klein wird.

14. Antrieb (200) nach Anspruch 13, wobei die Magnetkraft die Federkraft überwindet, wenn sich der Anker (204) zum ersten der U-förmigen Kerne (202) bewegt, so dass der Anker (204) in der eingezogenen Position magnetisch verriegelt ist.

15. Antrieb (200) nach Anspruch 1, wobei sich die Dicke der Polschuhe (228) an ihren freien Kanten auf Null verjüngt.

16. Ventrikuläre Hilfsvorrichtung mit einem oder zwei Beuteln (230) und einem Antrieb (200) nach Anspruch 1.

17. Antrieb (200) nach Anspruch 1,
wobei die Geometrie des Antriebs auf der Grundlage eines Modells, das die Leistungsziele für den Antrieb, ein empirisches Pumpen-Hydraulikdruck-Modell und Pumpenparameter zu einer Kraft-zu-Hub-Relation kombiniert, optimiert ist.

**Revendications**

1. Commande électromagnétique (200) comprenant :

    une paire de noyaux en forme de U (202), chacun ayant une section centrale (212) et deux jambes ;

une ou plusieurs bobines (220, 222, 224, 226), chaque bobine enroulée autour d'un noyau sélectionné parmi les noyaux en forme de U (202) qui, quand elles sont sous tension électrique,

génèrent un flux magnétique et définissent une ou plusieurs paires de pôles magnétiques ;

une armature (204) entre les noyaux en forme de U (202), ayant deux extrémités n'étant pas magnétiques permanentes (206, 208) et un aimant permanent (210) entre celles-ci, ledit aimant (210) pour générer une force d'aimant sur ladite armature (204) résultant de l'attraction dudit aimant (210) par lesdits noyaux en forme de U (202) quand lesdites bobines (220, 222, 224, 226) ne sont pas sous tension électrique ; dans laquelle lesdites une ou plusieurs bobines sous tension génèrent une force de bobine sur ladite armature qui est approximativement indépendante de la position de ladite armature et qui varie selon le degré de mise sous tension desdites bobines ; et

**caractérisée par** deux paires d'épanouissements polaires (228), chaque épanouissement polaire (228) couplé à une extrémité d'une jambe des noyaux en forme de U (202), alors que les épanouissements polaires (228) sont plus larges que les jambes des noyaux en forme de U (202).

2. Commande (200) selon la revendication 1, dans laquelle ladite commande a deux dites bobines (220, 222, 224, 226) chacune enroulée autour d'une section centrale de noyau en forme de U respective (212).

3. Commande (200) selon la revendication 1, dans laquelle ladite commande a une seule desdites bobines (220, 222, 224, 226), ladite bobine (220, 222, 224, 226) enroulée autour d'une section centrale sélectionnée parmi lesdites sections centrales de noyaux en forme de U (212), dans laquelle l'autre desdits noyaux en forme de U (202) est dépourvu de bobine.

4. Commande (200) selon la revendication 1, dans laquelle ladite commande a deux paires desdites bobines (220, 222, 224, 226) avec chacune desdites bobines (220, 222, 224, 226) enroulée autour d'une jambe respective desdits noyaux en forme de U (202) et chacune desdites paires ayant deux bobines adjacentes (220, 222, 224, 226).

5. Commande (200) selon la revendication 1, dans laquelle ladite commande a une paire desdites bobines (220, 222, 224, 226) avec chacune desdites bobines (220, 222, 224, 226) enroulée autour d'une jambe respective d'un desdits noyaux en forme de U (202), dans laquelle l'autre desdits noyaux en forme de U (202) est dépourvu de bobine.

6. Commande (200) selon la revendication 1, dans laquelle lesdites une ou plusieurs bobines (220, 222, 224, 226) génèrent une composante de flux qui passe de manière transversale à travers ladite armature (204).

7. Commande (200) selon la revendication 1, dans laquelle lesdites extrémités n'étant pas magnétiques permanentes (206, 208) de ladite armature (204) sont effilées de manière à réduire davantage la taille de ladite commande.

8. Commande (200) selon la revendication 1, dans laquelle une ou plusieurs paires d'espaces sont définies entre ladite armature (204) et chacune desdites une ou plusieurs paires de pôles magnétiques, et ladite commande ayant des bords de pôle étroits afin de réduire la perméance à l'effet de frange de ladite commande.

9. Commande (200) selon la revendication 1, dans laquelle ladite commande a des pôles de stator situés au niveau des extrémités desdites jambes dudit noyau en forme de U (202).

10. Commande (200) selon la revendication 1, comprenant en outre un cadre et un ou plusieurs ressorts (232) positionnés entre ledit cadre et ladite armature (204) de manière à exercer une force de ressort sur ladite armature.

11. Commande (200) selon la revendication 10, dans laquelle la somme de ladite force de ressort et de ladite force magnétique est d'environ zéro et est approximativement indépendante de la position de ladite armature (204).

12. Commande (200) selon la revendication 10, dans laquelle la somme de ladite force de ressort et de ladite force magnétique est une force de sollicitation nette qui est approximativement indépendante de la position de ladite armature (204) et sollicite ladite armature (204) vers une de ladite paire de pôles, de sorte qu'une force d'assistance d'expulsion constante est obtenue en décalant lesdits ressorts (232).

13. Commande (200) selon la revendication 10, dans laquelle ladite armature (204) est maintenue dans une position rétractée vers un premier desdits noyaux en forme de U (202) et contre ladite force d'assistance d'expulsion desdits ressorts (232) uniquement par ladite force d'aimant substantiellement sans mise sous tension desdites une ou plusieurs bobines (220, 222, 224, 226) en ayant un espace entre ladite armature (204) et lesdits noyaux en forme de U (202) deviennent substantiellement petits dans ladite position rétractée.

14. Commande (200) selon la revendication 13, dans laquelle ladite force d'aimant dépasse ladite force de ressort lorsque ladite armature (204) se déplace vers le premier desdits noyaux en forme de U (202) de sorte que ladite armature (204) est verrouillée mécaniquement dans ladite position rétractée.

15. Commande (200) selon la revendication 1, dans laquelle l'épaisseur des épanouissements polaires (228) s'effile jusqu'à une épaisseur nulle au niveau de leurs bords libres.

16. Dispositif d'assistance ventriculaire ayant un ou deux sacs (230) et une commande (200) selon la revendication 1.

17. Commande (200) selon la revendication 1, dans laquelle la géométrie de ladite commande est optimisée sur la base d'un modèle qui combine des cibles de performance pour ladite commande, un modèle de pression hydraulique de pompe empirique, et des paramètres de pompe en une relation force contre course.

FIG. 1 PRIOR ART

FIG. 2A
PRIOR ART

FIG. 2B
PRIOR ART

FIG. 2C
PRIOR ART

FIG. 2D
PRIOR ART

FIG. 2E
PRIOR ART

FIG. 2F
PRIOR ART

**FIG. 3**

Pump Center Section

**FIG. 4**

300

Flux Density B (T)

1.0

0.9

0.8

0.7

0.6

0.5

0.4

0.3

0.2

0.1

0.0

**FIG. 5**

**FIG. 6**

**FIG. 7**

16

FIG. 8

FIG. 9

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6264601 B
- DE 3804768